# EUROPEAN PATENT SPECIFICATION

(11) **EP 2 338 407 B1**
(45) Date of publication and mention of the grant of the patent: **12.02.2014**
(21) Application number: 09015952.6
(22) Date of filing: 23.12.2009
(51) Int. Cl.: A61B 3/14

(54) **Device for visually examining the ocular fundus of a patient**
Vorrichtung zur visuellen Untersuchung des Augenhintergrundes eines Patienten
Dispositif d'examen visuel du fond d'oeil d'un patient

(43) Date of publication of application: 29.06.2011
(73) Proprietor: OPTOPOL Technology Spolka Akcyjna, 42-400 Zawiercie (PL)
(72) Inventor: Carl, Thomas, Dr., 80333 München (DE)
(74) Representative: Rösler, Uwe

(56) References cited:
- EP-A1- 1 806 092
- GB-A- 1 401 664
- JP-A- 2001 008 899
- US-A- 4 732 466
- US-A- 5 321 446
- US-A1- 2006 146 401
- US-A1- 2007 291 277
- US-A1- 2008 079 901

## Description

### Technical Field

The invention relates to a device for visually examining the ocular fundus of a patient in accordance with the preamble of claim 1.

### The prior art

The present invention relates to a device for examining the ocular fundus of a patient and is used for example, for retinal examinations. It is necessary to prepare high-resolution, high-contrast colour or black and white images of the ocular fundus for such examinations in a continuous sequence. This is done on the one hand, in order to carry out a reliable diagnosis of the eyes, and on the other, to satisfy the requirements for documentation and the application of therapeutic measures. Here, the mapping of the ocular fundus represents a visual challenge, as both the illumination as well as the observation of the ocular fundus has be performed through a relatively small entrance pupil of the eye. In addition, the ocular fundus usually only exhibits a weak reflectivity which dominates for red colour components in such a way that high-contrasting colour images of the ocular fundus can usually only be produced with one light source with strong blue and green components. The visual examination of the ocular fundus with the aid of a light beam (which is examined as an observation beam following reflection e.g. on the retina) is additionally hampered by other unwanted reflections at the interfaces of the cornea as well as by unwanted light scattering, e.g. at vitreous bodies.

Disclosed in EP 1389943 B1, the generic device of a oscillating shutter system solves the scattering problem in the following manner. The device comprises the following components: an illumination device for generating a light beam; an illumination projection lens for mapping the light beam onto the eye, a means for scanning the light beam across the eye; a observation device; an observation projection lens for mapping a observation beam onto the observation device which is generated by the reflection of the light beam on the eye, as well as a means for blocking out light that is scattered from the observation beam.

A halogen lamp is used as a illumination device, the light of which is collimated by a condenser and is focused onto the oculus fundus of the patient via the illumination projection lens. The observation beam reflected by the ocular fundus is mapped via the observation projection lens onto an image plane where there is a CCD sensor as an observation device. In this generic device, the means for scanning the light beam over the eye is realised by a slit shutter which oscillates in front of the halogen lamp in the light beam collimated from the condenser. This shutter is made from an opaque material, e.g. a flat sheet material, and only allows through a linear section of the light beam as defined by the size of the slit shutter, which, due to the oscillation of the slit shutter, is similarly scanned back and forth over the eye of the patient with this oscillation frequency.

In this known device, the means for blocking out light which is scattered from the observation beam are also formed by a mechanically oscillating slit shutter. In particular, EP 1389943 B1 suggests forming slit shutter oscillating in front of the halogen lamp and the slit shutter oscillating in front of the CCD (which, for reasons of the mapping procedure, have to be synchronised with each other anyway) as a pair of slit shutters in a single flat sheet.

In practice, it has been shown here that the selection of a system with a slit shutter oscillating back and forth in front of the sensor in particular leads to problems. On the one hand, the oscillations from the mechanical oscillator cannot be sufficiently separated from the housing of the device. This can reduce the imaging sharpness of the sensor due to vibrations.

In US 4,732,466, a scanning ocular fundus camera with an light beam path and an observation beam path is described which envisages a rotating drum with two adjacent rows of slits within the drum wall respectively. As such, the known arrangement avoids the above-described vibration problem, but has a large structural shape and moreover, allows no or very limited optical variations, such as the injection of other beams of light etc., in order, for example, to additionally inject a therapy beam path into the observation optics of the fundus camera used for diagnosing the ocular fundus. In addition, using the known arrangement, it is not possible, or only possible with a large amount of additional assembly work, to adapt the area on the ocular fundus, which is specified, illuminated and at the same time observable through the slit size, to different diagnostic situations. For carrying out angiographic examinations of the ocular fundus, a pupil that is as dilated as possible is required, a so-called mydriatic pupil through which an illumination beam chosen to be large as possible in the beam cross section can pass in order to illuminate and at the same time observe as large an area of the ocular fundus as possible. On the other hand, in the case of so-called non-mydriatic eye examinations, i.e. with a narrow pupil, the illumination and observation of the ocular fundus needs to be adapted to the geometric conditions of pupil in order to obtain an image of usable quality.

The desire for maximum variability in terms of the options for optical examination, as well as the demand for high quality images of the ocular fundus also leads to the combination of a such a generic ophthalmoscopic arrangement with other diagnostic and/or treatment systems for examining and treating the eye.

In this context, the ability to combine the technique applied in the eye examination process for generating images of a cross section through the ocular fundus, in short, the OCT method (ocular coherence tomography), with a high quality imaging system for representing the ocular fundus is particularly important.

With highly technical and elaborate equipment, the OCT technique enables in vivo-sectional images through the ocular fundus. With this, the smallest retinal structures can be represented as a sectional view. This examination technique allows complete in-vivo measurement of the optic nerve head and the peripapillary nerve fibre layer, something which is currently impossible with other imaging techniques.
As such, ocular coherence tomography provides the foundation for objective observation relating to the course of various eye diseases, especially for glaucoma.

A largely simultaneous means for visually observing the ocular fundus would be very helpful, especially for improved positioning / recording of OCT-sectional images and a better diagnosis with the aid of OCT-sectional images through the ocular fundus. As such, the positioning of individual OCT sectional photographs could be carried out with regard to a colour or black and white image of the ocular fundus which has been taken a short while directly beforehand.

US 5,321,446 discloses a slit lamp microscope for analyzing the fundus of an eye which additionally can be operated in a mode for inspection of different defined depth sections of the eye. The slit lamp microscope hereto comprises a diaphragm disc in form of a rotating Nipkow disk through which the illumination and observation beam of the microscope pass. For inspection the fundus of the eye the pivotable slit lamp is in operation but during observation of different defined depth sections of the eye an extra light source is provided while the slit lamp is turned off.

### Summary of the invention

It is therefore an object of the invention to further develop a generic device for visually examining the ocular fundus of a patient in such a way that disturbing influences which affect the image quality of the photographs of the ocular fundus, e.g. scattered light, vibrations, etc., are avoided. The device should be designed so as to be as compact as possible with few components, however it should offer the opportunity to inject additional beam paths, e.g. radiation therapy beams, into the existing optical paths along the lighting and/or observation beam path, without compromising them. The device should possess a high degree of variability in relation to carrying out eye examinations on mydriatic or non-mydriatic pupils. Finally, the device for visually examining the ocular fundus of a patient should be capable of being integrated or combined with a device for carrying out ocular coherence tomography in such a way that the beam paths of both appliances do not adversely affect each other.

The solution of the task which forms the basis of the invention is specified in claim 1. Further advantageous features in accordance with the idea of the invention form the subject of the subclaims as well as the further description, especially with reference to the example embodiments.

In accordance with the solution, the device for visually examining the ocular fundus of a patient with the features of the preamble of claim 1 is characterised by the fact that a roof-edge prism is provided along the light beam mapped to infinity and a deflecting element is provided along the observation beam mapped to infinity wherein at least one of the two, i.e. the roof-edge prism and/or the deflecting element are arranged variably in relation to one another in terms of location so that an assignable lateral beam displacement and accordingly pupillary distance can be varied between the illumination and observation beam within the second mapping objective. In addition, the means for scanning the light beam and the means for blocking out scattered light can be converted reversibly between at least two operating positions in such a way that in a first operating position of the light and observation beam on the ocular fundus has a first cross-sectional area and in a second operating position of the light and observation beam on the ocular fundus has a second cross-sectional area, wherein the first and second beam cross-sectional areas are different.

This way for example, it is advantageous when conducting eye examinations with a narrow pupil, i.e. a non-mydriatic pupil, to select the lateral beam displacement between the light beam and the observation beam to be as small as possible in order to map the light beam as well as the observation beam into the eye without lateral border shadowing through the already very narrow pupil size.
Such eye examinations are usually done for taking colour or black and white photographs of the ocular fundus, where for high-contrast imaging of the ocular fundus, sufficient light is available for its illumination, e.g. from a light source formed from a halogen lamp. In this case, the means for scanning the light beam and the means for blocking out scattered light are in an operating position in which the light beam and the observation beam, produce a smaller cross-sectional area on the ocular fundus.

The situation is different however, when taking angiographic photographs for which only a very limited frequency spectrum is used in a targeted way, to stimulate the fluorescence of a contrast agent which has been administered to the patient. In this case, preferably only one green-blue light component, (limited spectrally by filters) reaches the ocular fundus from the light source, so that for illuminating the ocular fundus, the means for scanning the light beam and the means for blocking out stray light are brought into an operating position in which the light beam and the observation beam generate the widest possible cross-sectional area on the ocular fundus. Such image representations of the ocular fundus however, need a pupil which is as wide as possible, over which a largest proportion of light can be applied to the ocular fundus as possible. In this case, it is advantageous to select the lateral beam displacement between the light beam and the observation beam to be as large as possible, so as to avoid disturbing reflections on the eye. As the other embodiments will show, in particular with reference to the relevant exemplary embodiment, only a combined linear displacement of the roof-edge prism and the deflecting element is necessary in order to variably adjust each lateral beam displacement between the light beam and the observation beam in such a way that the lateral beam displacement of the light beam and the observation beam is chosen respectively of equal size with respect to a common optical axis.

In a preferred embodiment, both the means for scanning the light beam and the means for blocking out scattered light along the observation beam is realised in the form of a drum fitted with slits, which rotate at a constant speed around an axis of rotation.

In a preferred embodiment variant of the slit drum, the drum has two rows of slits which are arranged side by side in the circumferential direction of the drum, wherein the slits are respectively dimensioned within a row of slits so that they are the same size, however the slit sizes of each different row of slits are different, and particularly exhibit different slit widths. As the slit drum is mounted such that it is axially displaced longitudinally along its axis of rotation, as will be explained in further detail, differently sized slits can be selected according to the eye examination mode. If for example, an angiographic examination of the ocular fundus is to be performed as has already been mentioned above, with light from a spectrally limited frequency spectrum for the purposes of using targeted fluorescence stimulation, each row of slits in the path of the light beam and the observation beam will be inserted where the slit width is measured as greater, whereby for each illumination slit, more light can pass through. Further details are reserved for the description with reference to the corresponding example embodiment.

A particularly advantageous further development of the device in accordance with the solution, for visually examining the ocular fundus is provided by a novel combination with a device for ocular coherence tomography (in short, OCT) which in order to record cross-sectional images of structures depicting the ocular fundus, is provided with a mapping lens for mapping a measuring beam in the area of the ocular fundus, via which, the measuring beam which is scattered and/or reflected onto the ocular fundus is also mapped backwards into the OCT for further recording and analysis of the measuring beam's scattered components. The proposed combination of SRC and OCT shares the ophthalmoscopic lens unit belonging to the SRC for the respective beam mapping. This presupposes however, that the imaging lens of the OCT has a compensating lens, so that the mapping conditions of the OCT, which otherwise remain unchanged, are preserved. As such, the mapping lens of a conventional OCT device has a focal range which, in the above-mentioned combination scenario, corresponds to the resulting focal range of an optical system consisting of the ophthalmoscopic lens unit and the compensation lens.

In addition, for the systemic combination of the individual beam paths of the OCT and the SRC, a beam combination unit needs to be introduced between the ophthalmoscopic lens unit and compensating lens, which combines the measuring beam of the OCT and the light beam and observation beam of the SRC for jointly mapping through the ophthalmoscopic lens unit. A factor which is very significant here is that the beam paths for the OCT as well as for the SRC do not interfere with each other. In a particularly advantageous arrangement, a geometric or dichroic beam splitter is suitable for the beam combination unit.

Through such a combination of two eye examination devices which up until now, have been used as a stand-alone units, it is possible to optimally evaluate cross-sectional images of the ocular fundus (which have been recorded using OCT technology) through largely simultaneously recorded images of the ocular fundus, e.g. within the scope of so-called colour photography of the ocular fundus or angiographic image representation. As such, on the basis of such images of the ocular fundus, the cross-section images which have been gained using OCT technology can be accurately positioned.

### A short description of the invention

In the following, the invention will be described by means of example embodiments with reference to the figures without restricting the general inventive concept. The following is shown:
- Fig. 1: A representation of the components of a device designed in accordance with the solution for visually examining of the oculus fundus of a patient.
- Fig. 2: A schematic outline sketch for combining a device in according with Figure 1 with a device for ocular coherence tomography.
- Fig. 3: A detailed example embodiment for the combination described in Figure 2.

### Ways for designing the invention, industrial applicability

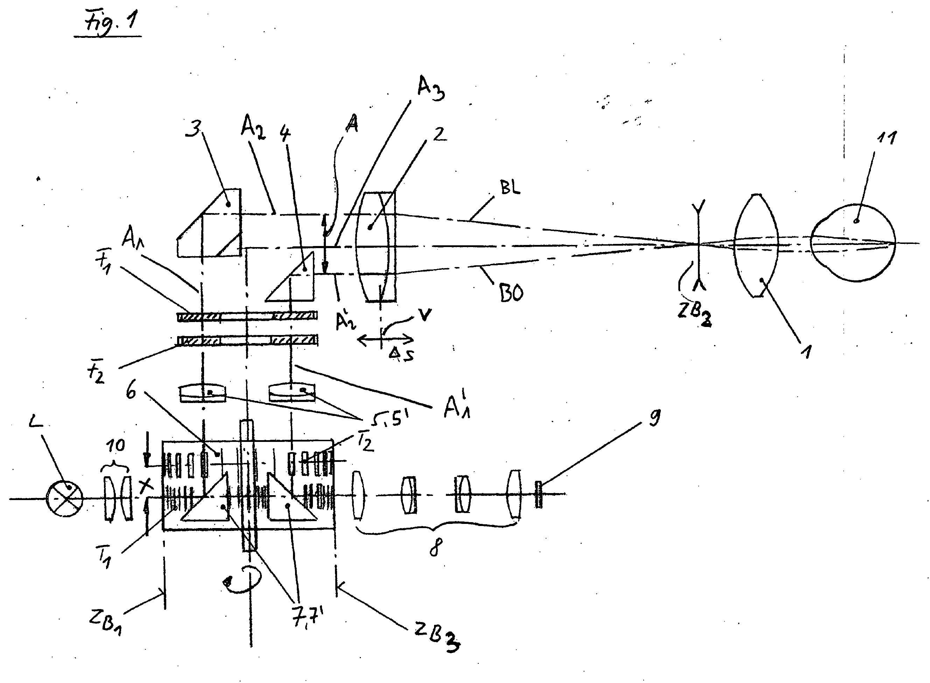

Figure 1 shows the light beam path as well as the observation beam path relating to a device for visually examining the ocular fundus including all the important components. The principle is to sharply map the light beam BL emanating from a light source L onto the ocular fundus of an eye 11 in a visual way. Furthermore, the principle is to visually map the light scattered onto the ocular fundus of the eye 11 in the form of an observation beam BO on an observation device 9, e.g. in the form of a CCD camera. In a way which is known, a scanning illumination and observation technique is used in which the ocular fundus of the patient's eye 11 is illuminated in strip-like shapes. This is done in order to obtain the images of the ocular fundus, which are recorded by means of the observation device 9, in a way which provides as much contrast as possible and which is free from the reflection and scattering effects occurring in the eye 11. For this, the light beam BL emerging from the light source L is mapped via a condenser lens 10 into a intermediate image plane ZB₁ in which a drum 6 is arranged which is provided with slits T1. The drum 6 is swivel mounted around an axis of rotation R, and preferably rotates at a constant rotational speed so that oscillations are wholly avoided. The slits T1 arranged along the first row of slits have a uniform size and as such, a uniform slit width. During the rotation around the axis of rotation R, they pass through the homogeneously mapped light beam BL in the intermediate image plane ZB₁.

The light beam BL which is broken by the rotation of the drum 6 in time-sequential slit images encounters a deflecting unit 7 arranged inside the drum 6, e.g. in the form of a full mirror or prism, which deflects the light beam BL by 90 ° longitudinally to the optical axis A1 into a first mapping objective 5 which maps the relevant strip-shaped light beam into infinity, i.e. converts it into a parallel split bundle of beams. A roof-edge prism 3 is provided along the optical axis A1 which on the one hand, deflects the light beam BL by 90 ° and on the other, maps to form a reversed image, i.e. in a rotation of the drum 6 as implied Figure 1, which moves vertically from above in the area of the intermediate image plane ZB1 in the direction of the plane, the individual slit images, each respectively extending from top to bottom, are reversed in their direction of motion after passing through the roof-edge prism 3, i.e. the slit images move in the area along the optical axis A2 from the bottom upwards.
This action ultimately results in a synchronisation motion between the light beam BL and the observation beam BO at the point where the observation device 9 is established, as will be further explained in the following.

Following the light beam BL, a second mapping objective 2 is provided which maps light beam BL mapped into infinity into a second intermediate image plane ZB2, from which a ophthalmoscopic lens unit 1 is able to sharply map the light beam BL on the curved ocular fundus of the eye 11. For this, the ophthalmoscopic lens unit 1 has an aspherical surface, through which the plane intermediate image ZB2 is sharply mapped on the individually curved ocular fundus of the eye 11.

On the ocular fundus 11, the sharply mapped slit image of the light beam BL is scattered so that the scattered light components which pass through the ophthalmoscopic lens unit 1 and form the path of the observation beam BO are sharply mapped in turn in the intermediate image plane ZB2.
The second mapping objective 2 is able to map the intermediate image ZB2 assigned to the observation beam BO in the opposite direction into infinity. The objective 2 which does the mapping has a lens diameter, which is selected to be large enough so that both the light beam BL and the observation beam BO are mappable by the mapping objective 2, an measure that contributes to the compact design of the device.

A deflection unit 4, preferably in the form of a full mirror or a prism, directs the observation beam BO along an optical axis A1' which is oriented parallel to the optical axis A1 in the direction of another mapping objective 5', which is formed separately to the mapping objective 5 and ultimately maps the observation beam BO via a deflecting mirror 7' to an intermediate image plane ZB3 in which the slit T1 of the slit drum 6 rotate. With the aid of a mapping lens 8, preferably a 1:1 mapping lens, the observation beam BO is mapped from the intermediate image plane ZB3 to the observation device 9 formed as a CCD camera for visual recording.

The device for visually examining the ocular fundus of a patient which can be seen in Figure 1 (in short, SRC) uses the slit T1 of a single row of slits of the drum 6 for the scanning light as well as the observation, wherein the places where the light beam BL as well as the observation beam BO passes through the row of slits are at two different locations in the circumferential direction of the drum 6, preferably, as can be seen in Figure 1, at opposite locations diametric to the axis of rotation R. On the one hand, such a structure makes it possible to construct a very compact and technically easy, realisable design. On the other hand, any vibration problems, as is known about from the oscillating shutters, are avoided due to the uniform rotation of the drum around the rotational axis R.

Furthermore, the roof-edge prism 3 and the deflecting unit 4 are linearly, movably mounted along the optical axes A1 and A1' so that the beam axis A2 of the light beam BL and the beam axis A2 ' of the observation beam BO, can at least be variably moved in the range of the mapping objective 2 relative to the optical axis A3 of the second mapping objective 2. If, for example, a measurement is to be made on a narrow pupil, the pupillary distance A and/or the lateral beam displacement from the light beam and observation beam relative to the optical axis A3 should be chosen to be as small as possible. For this, the roof-edge prism 3 as well as the deflecting unit 4 are moved as close to the optical axis A3 as possible. On the other hand, a large pupillary distance A and/or a large lateral beam displacement of the beam axes A2 of the light beam and/or A2' of the observation beam relative to the optical axis A3, enables measurement of the ocular fundus with a widened pupil, i.e. in the case of a mydriatic pupil. By varying the pupillary distance A between the light beam and the observation beam , the angle which includes the light beam and the observation beam of the eye 11 can above all be varied, whereby distracting reflections which occur in the eye can be avoided through an appropriate choice of angles.

As the deflecting unit 4 as is shown in Figure 1, is arranged along the optical axis A3 between the roof-edge prism 3 and the mapping objective 2, it is possible to shadow the light beam BL by the deflecting unit 4 by selecting the appropriate size of the deflector 4, and by falling short of a certain reduced pupillary distance A. Such shadowing ultimately influences the amount of light which can be shed on the ocular fundus, the variation of which can also be used in a suitable way for optimised viewing of the ocular fundus.

In addition to the first row of slits which has already been discussed, the drum 6 has a second row of slits (with slits) T2, each with a slit width which is chosen to be greater than the slit width of slit T1. Through an axial shift in position along the drum's 6 axis of rotation R, slit T2 can be introduced to the path of the light beam and observation beam, through which, due to the larger gap geometry, more light can be shed on the ocular fundus. The larger slits T2 are used to carry out angiographic examinations of the ocular fundus, especially as only a portion of the light spectrum made available by a halogen lamp as a light source is normally used to produce fluorescence stimulation on the ocular fundus. In the case of an angiographic examination, at least one pivotally mounted filter F1 is introduced into the path of the light beam and the observation beam. In each case, this is done between the mapping objectives 5 and 5', the roof-edge prism 3 and the deflecting unit 4.

The filter arrangement F1 is, for example, only transparent to light with a wavelength of less than 500 nm. In order to map the light beam which, in this case, has been reduced in intensity, over an area on the ocular fundus of the eye 11 which is as large as possible, the roof-edge prism 3 and the deflecting unit 4 are set with the largest possible pupilliary distance, especially as in this case, the pupil of the eye 11 is widened through the use of drugs.

Likewise, using the device shown in Figure 1, it is possible to display a non-mydriatic ocular fundus in the proximal infrared range. For this, instead of filter F1, a second filter arrangement F2 (which is intended to be pivoted) is pivoted into the path of the light beam and observation beam. This arrangement will only allow passage of parts of the spectrum in the in the near infrared range.

Finally, in order to allow for compensation of errors when mapping the eyes, the mapping objective 2 is mounted so that it can be moved along the optical axis A3 via an adjustment path V. This is done in order to compensate mapping errors due to the eyes and to ensure a sharp mapping of the slit image onto the ocular fundus.

As such, the described SRC with a drum having two rows of slits as well as the varying the pupillary distance between the light beam observation beam enables individual adjustment of the slit image size and light intensity, depending in each case on the selected type of optical eye examination.

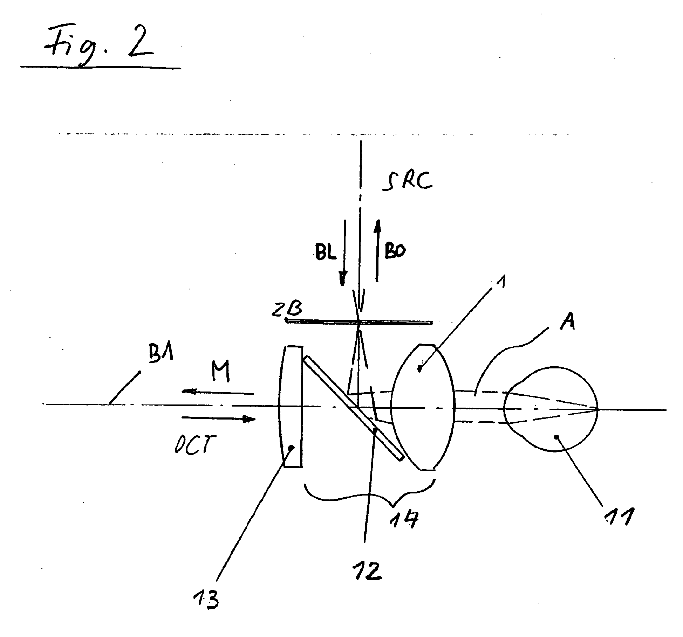

In addition, the SRC which has been discussed is advantageously suited for combining with a device for carrying out ocular coherence tomography (in short, OCT) as can be seen, for example, in EP 1 870 030 A1. Such OCT is suited for taking photographs of a cross-section image along a selectable sectional plane through the ocular fundus. A measuring beam is projected into the eye which is mapped onto the ocular fundus via an mapping lens from the OCT. For the purposes of developing a combination of the above-described SRC device and OCT, a solution is proposed for mapping the OCT measuring beam as well as the light beam and observation beam of the SRC, using the aspheric ophthalmoscopic lens unit which is jointly designed for the path of the SRC's light beam and observation beam. Figure 2 shows a structure for such a combination of the two devices. It is assumed that the measuring beam of the OCT is oriented along the optical axis B1 and the aspheric ophthalmoscopic lens unit is mapped into the eye 11 via the so-called compensating lenses 13. The compensating lenses 13 ensure an OCT-side, focal width adjustment of the measuring beam M, starting from the visual characteristics of the aspherical ophthalmoscopic lens unit 1. A beam combination unit 12 is introduced between the compensation lens 13 and the aspherical ophthalmoscopic lens 1, preferably in the form of a geometrically or dichroically design beam splitter, which is transparently formed for the measuring beam M of the OCT. A narrow-band light with a wavelength of around 820 nm is mostly selected for the OCT measuring beam M, so that beam combination unit 12 which is preferably formed as a dichroic beam splitter is transparent, corresponding to this wavelength range.

On the other hand, the beam combination unit 12 is formed to be highly reflective for both the light beam as well as the observation beam of the SRC beam, so that the beam combination unit of both devices (illustrated in Figure 2) can be realised.

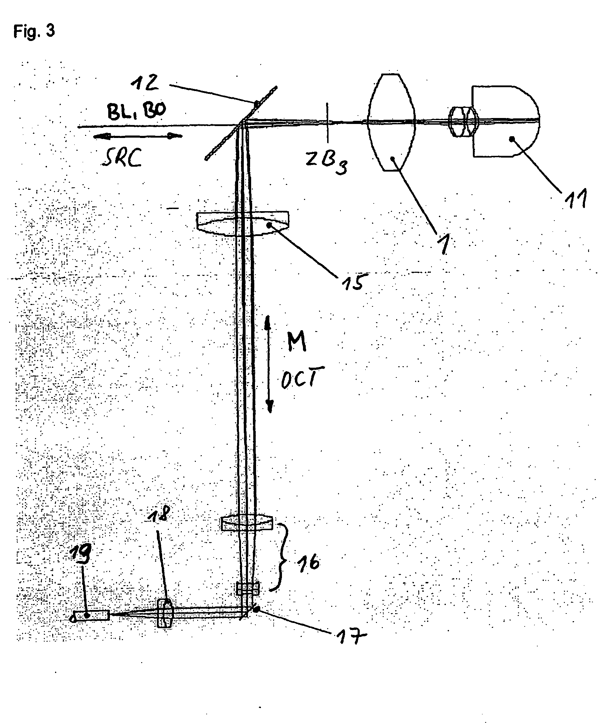

In Figure 3, a further alternative to the OCT and SRC beam combination is shown in which the light beam and observation beam BL, BO, largely pass without loss through the beam combination unit 12 and are mapped on the ophthalmoscopic lens unit 1 on the ocular fundus of the eye 11. In contrast, the OCT measuring beam M is deflected at the beam combination unit 12 in a way which is highly reflective. In particular, it is assumed in this case that the OCT measuring beam M decouples from an optical fibre 19 and is transferred through a telecentrically arranged input lens 18 into an expanded beam, which passes through a scanning mirror 17 into a Galilean afocal system 16 and after this, a focusing achromatic lens 15. This maps the measuring beam M via the beam combination unit 12 into an intermediate image plane ZB3, from which the aspherical ophthalmoscopic lens unit 1 maps the light beam onto the retina and/or the ocular fundus of the eye.

As such, the joint use of the aspherical ophthalmoscopic lens unit 1 is of crucial importance, both for the path of the light beam and observation beam relating to the SRC as well as for the measuring beam path of the OCT.

### List of reference signs

- 1: Ophthalmoscopic lens
- 2: Mapping objective
- 3: Roof-edge prism/Mirror
- 4: Deflecting mirror/Prism
- 5, 5': Mapping objective for illumination and observation
- 6: Rotation drum
- 7: Deflection mirror (for illumination and observation)
- 8: Mapping lens (for camera)
- 9: Observation device
- 10: Condenser
- 11: Eye
- 12: Beam combining unit, dichroic mirror
- 13: Compensations lens
- 15: Focusing achromatic lens
- 16: Afocal Galilean lens
- 17: Scanning mirror
- 18: Telecentric input lens
- 19: Fibre optic
- L: Light source
- T1: Narrow slit
- T2: Wide slit
- X: Movement path for the slit drum
- ZB 1,2,3: Intermediate image planes
- A: Pupillar distance between the light beam and the observation beam
- F1, F2: Filter

## Claims

1. A device for visually examining the ocular fundus of a patient with
- an illumination device (L) for generating a light beam;
- an illumination-mapping optics for projecting the light beam onto the eye, containing a condenser lens (10) mapping a light beam emerging from the illumination device (L) in a intermediate image plane (ZB1), a first mapping objective (2) which maps the intermediate image plane (ZB1) into infinity, a second mapping objective (5) mapping the intermediate image plane which is mapped into infinity onto a second intermediate image plane (ZB2) as well as a ophthalmoscopic lens unit (1) mapping the second intermediate image plane (ZB2) onto the ocular fundus,
- a means for scanning the illumination beam across the eye (11),
- an observation device (9);
- an observation-mapping optics for mapping a observation beam on the observation device (9) generated on the eye (11) by the reflection of the light beam comprising the light beam reflected on the ocular fundus into the ophthalmoscopic lens unit (1) mapping the second intermediate image plane (ZB2), the second mapping objective (2) which maps the second intermediate image plane (ZB2) into infinity, an observation beam mapped into infinity in a third intermediate image plane (ZB3) by mapping objective (5'), and a third mapping lens (8) mapping the third intermediate image plane (ZB3) into the observation device (9), and
- a means for blocking out scattered light from the observation beam, **characterised in that** a roof-edge prism (3) is provided along the illumination beam which is mapped into infinity and a deflecting element (4) is provided along the observation beam which is mapped into infinity, and
that the roof-edge prism (3) is arranged such that its position is changeable and/or the deflecting element (4) is arranged such that its position is changeable, in such a way that an assignable lateral beam displacement (A) is variable between the illumination beam and the observation beam within the second mapping objective (2), and
that the means for scanning the illumination beam and the means for blocking out scattered light reversibly are convertible between at least two operating positions in such a way that in a first operating position of the illumination and observation beam on the ocular fundus has a first beam cross-sectional area and in a second operating position of the light and observation beam on the ocular fundus has a second beam cross-sectional area, wherein the first and second beam cross-sectional areas are different.

2. The device in accordance with claim 1,
**characterised in that** the means for scanning the illumination beam and the means for blocking out scattered light are formed as a drum (6) rotating around an axis which has at least a first and a second row of slits (T1, T2), in which respectively a number n ≥ 2 of equally dimensioned slits is provided with in each case, a paired equidistant distance in the circumfrential direction of the drum (6),
- that the illumination beam and the observation beam passes through the row of slits (T1, T2) are at two different locations in the circumferential direction of the drum (6),
- that the second row of slits (T2) is arranged longitudinally with respect to the rotating axis next to the first row of slits (T1) in the drum (6),
- that the slit in the second row of slits (T2) has a different size from the slits in the first row of slits (T1), and
- that relative to the observation beam and the illumination beam, the drum (6) is changeable in terms of its location.

3. The device in accordance with claim 2,
**characterised in that** the slits (T1, T2) have a slit length oriented parallel to the axis of rotation and a slit width oriented vertically to the axis of rotation and
that the slits of the second row of slits (T2) have a larger slit width than the slits of the first row of slits (T1).

4. The device according to any on of claims 1 to 3,
**characterised in that** along an optical axis assigned to the second mapping objective (2), the deflecting element (4) is arranged between the roof-edge prism (3) and the second imaging objective (2), and
that the illumination beam and the observation beam, at least exhibit an equally sized lateral beam displacement (A) of the optical axis in the area between the deflector element (4) and the second imaging objective (2).

5. The device according to any one of claims 2 to 4,
**characterised in that** the places at which the illumination beam and the observation beam pass through the at least one row of slits, are diametrically opposite each other in the circumferential direction of the drum (6).

6. The device according to any one of claims 2 to 5,
**characterised in that** the first intermediate image plane (ZB1) is located at the place at which the illumination beam traverses the row of slits of the drum (6), and that the third intermediate image plane (ZB3) is located at the point at which the observation beam passes through the row of slits of the drum (6).

7. The device according to any one of claims 2 to 6,
**characterised in that** within the drum (6), a beam deflecting element (7) is provided for the illumination beam and another beam deflecting element (7') for the observation beam, where the mutual distance sets a maximum lateral beam displacement between the light beam and the observation beam.

8. The device according to any one of claims 1 to 7,
**characterised in that** in the area of the illumination beam and observation beam which are respectively mapped to infinity, at least one filter unit (F1, F2) is provided along the illumination beam and/ or observation beam that is formed and arranged such that it is pivotable and/or variable in its filtering effect.

9. The device according to any one of claims 1 to 8,
**characterised in that**, the ophthalmoscopic lens unit (1) is an aspheric lens unit which maps the second intermediate image plane (ZB2) onto the ocular fundus.

10. The device according to claim 9 in combination with a device for ocular coherence tomography (in short, OCT) which has an mapping lens for mapping a measuring beam into the area of the ocular fundus as well as for the reverse mapping of measuring beam components scattered on the ocular fundus,
**characterised in that** the mapping lens of the device for ocular coherence tomography (OCT) consists of the ophthalmoscopic lens unit (1) and a compensating lens (13), and
that a beam combination unit (12) is introduced between the ophthalmoscopic lens unit (1) and the compensating lens (13) which combines the measuring beam (M) and the light beam and observation beam to the common mapping through the ophthalmoscopic lens unit (1).

11. The device according to claim 10,
**characterised in that** the beam combination unit (12) is designed as a geometric or a dichroic beam splitter.

12. The device according to claim 11,
**characterised in that** the geometric or dichroic beamsplitter is transparent for the measuring beam and reflective for the light beam and observation beam, or vice versa.

13. The device according to any one of claims 10 to 12,
**characterised in that** the mapping lens of the OCT has a focal width which corresponds to the resulting focal length of an optical system consisting of ophthalmoscopic lens unit (1) and the compensation lens (13).

14. The device according to any one of claims 10 to 13,
**characterised in that** the compensation lens (13) consists of the following components:
a telecentric lens system (18) which maps the measuring beam into infinity, a Galilean afocal system (16) which maps the parallel oriented measuring beam into a focusing achromatic lens (15) which maps the measuring beam into the second intermediate image plane (ZB2).

15. The device according to claim 14
**characterised in that** the beam combination unit (12) is arranged between the focusing achromatic lens (15) and the second intermediate image plane (ZB2).

## Patentansprüche

1. Vorrichtung zur optischen Untersuchung des Augenhintergrundes eines Patienten mit
- einer Beleuchtungsvorrichtung (L) zur Erzeugung eines Beleuchtungsstrahls;
- einer Beleuchtungs-Abbildungsoptik zum Abbilden des Beleuchtungsstrahls auf das Auge, umfassend eine den aus der Beleuchtungsvorrichtung (L) austretenden Beleuchtungsstrahl in eine Zwischenbildebene (ZB1) abbildende Kondensoroptik (10), ein erstes Abbildungsobjektiv (2), das die Zwischenbildebene (ZB1) ins Unendliche abbildet, ein die ins Unendliche abgebildete Zwischenbildebene (ZB1) auf eine zweite Zwischenbildebene (ZB2) abbildendes zweites Abbildungsobjektiv (5) sowie eine die zweite Zwischenbildebene (ZB2) auf den Augenhintergrund abbildende Ophtalmoskopierlinseneinheit (1),
- Mittel zum Scannen des Beleuchtungsstrahls über das Auge (11),
- eine Beobachtungsvorrichtung (9);
- eine Beobachtungs-Abbildungsoptik zum Abbilden eines durch Reflexion des Beleuchtungsstrahls am Auge (11) erzeugten Beobachtungsstrahls auf die Beobachtungsvorrichtung (9), umfassend die den am Augenhintergrund reflektierten Beleuchtungsstrahl in die zweite Zwischenbildebene (ZB2) abbildende Ophthalmoskopierlinseneinheit (1), das die zweite Zwischenbildebene (ZB2) ins Unendliche abbildende zweite Abbildungsobjektiv (2), ein den ins Unendliche abgebildete Beobachtungsstrahl in eine dritte Zwischenbildebene (ZB3) abbildendes Abbildungsobjektiv (5') sowie eine die dritte Zwischenbildebene (ZB3) in die Beobachtungsvorrichtung (9) abbildende dritte Abbildungsoptik (8), sowie
- Mittel zum Ausblenden von Streulicht aus dem Beobachtungsstrahl,
**dadurch gekennzeichnet, dass** längs des ins Unendliche abgebildeten Beleuchtungsstrahls ein Dachkantprisma (3) und längs des ins Unendliche abgebildeten Beobachtungsstrahls eine Umlenkelement (4) vorgesehen sind, dass das Dachkantprisma(3) lageveränderlich angeordnet ist und/oder das Umlenkelement (4) lageveränderlich angeordnet ist, derart, dass ein zwischen dem Beleuchtungs- und Beobachtungsstrahl innerhalb des zweiten Abbildungsobjektivs (2) zuordenbarer lateraler Strahlversatz (A) variierbar ist, und
dass das Mittel zum Scannen des Beleuchtungsstrahls und das Mittel zum Ausblenden von Streulicht reversibel zwischen wenigstens zwei Betriebsstellung derart überführbar ist, dass in einer ersten Betriebsstellung der Beleuchtungs- und Beobachtungsstrahl am Augenhintergrund eine erste Strahlquerschnittsfläche und in einer zweiten Betriebsstellung der Beleuchtungs- und Beobachtungsstrahl am Augenhintergrund eine zweite Strahlquerschnittsfläche aufweist, wobei sich die erste und zweite Strahlquerschnittsflächen unterscheiden.

2. Vorrichtung nach Anspruch 1,
**dadurch gekennzeichnet, dass** das die Mittel zum Scannen des Beleuchtungsstrahls und das Mittel zum Ausblenden von Streulicht als eine, um eine Achse rotierende Trommel (6) ausgebildet ist, die über wenigstens eine erste und zweite Spaltreihe (T1, T2) verfügt, in denen jeweils in Umfangsrichtung der Trommel (6) eine Vielzahl n ≥ 2 gleich dimensionierte Spalte mit jeweils paarweise äquidistantem Abstand vorgesehen ist, dass der Beleuchtungs- und Beobachtungsstrahl die Spalte einer Spaltreihe (T1, T2) an zwei in Umfangsrichtung der Trommel (6) unterschiedlichen Orten durchsetzt, dass die zweite Spaltreihe (T2) längs zur rotierenden Achse neben der ersten Spaltreihe (T1) in der Trommel (6) angeordnet ist,
dass die Spalte in der zweiten Spaltreihe (T2) eine von den Spalten in der ersten Spaltreihe (T1) unterschiedliche Spaltgröße aufweisen, und
dass die Trommel (6) relativ zu dem Beobachtungs- und Beleuchtungsstrahl lageveränderlich ist.

3. Vorrichtung nach Anspruch 2,
**dadurch gekennzeichnet, dass** die Spalte eine parallel zur Rotationsachse orientierte Spaltlänge und eine senkrecht zur Rotationsachse orientierte Spaltbreite aufweisen, und
dass die Spalte der zweiten Spaltreihe (T2) eine größere Spaltbreite aufweisen als die Spalte der ersten Spaltreihe (T1).

4. Vorrichtung nach einem der Ansprüche 1 bis 3,
**dadurch gekennzeichnet, dass** längs zu einer dem zweite Abbildungsobjektiv (2) zuordenbaren optischen Achse das Umlenkelement (4) zwischen dem Dachkantprisma (3) und der zweiten Abbildungsobjektiv (2) angeordnet ist, und dass der Beleuchtungs- und der Beobachtungsstrahl zumindest im Bereich zwischen der Umlenkeinheit (4) und dem zweiten Abbildungsobjektiv (2) einen jeweils gleichgroßen lateralen Strahlversatz zur optischen Achse aufweisen.

5. Vorrichtung nach einem der Ansprüche 2 bis 4,
**dadurch gekennzeichnet, dass** die Orte, an denen der Beleuchtungs- und Beobachtungsstrahl die wenigstens eine Spaltreihe durchsetzen, sich in Umfangsrichtung der Trommel (6) diametral gegenüberliegen.

6. Vorrichtung nach einem der Ansprüche 2 bis 5,
**dadurch gekennzeichnet, dass** sich die erste Zwischenbildebene (ZB1) am Ort, an der der Beleuchtungsstrahl die Spaltreihe der Trommel (6) durchsetzt, befindet, und dass sich die dritte Zwischenbildebene (ZB3) am Ort, an der der Beobachtungsstrahl die Spaltreihe der Trommel (6) durchsetzt, befindet.

7. Vorrichtung nach einem der Ansprüche 2 bis 6,
**dadurch gekennzeichnet, dass** innerhalb der Trommel (6) ein Strahlumlenkelement (7) für den Beleuchtungsstrahl und ein weiteres Strahlumlenkelement (7') für den Beobachtungsstrahl vorgesehen sind, deren gegenseitiger Abstand einen maximalen lateralen Strahlversatz zwischen dem Beleuchtungs- und Beobachtungsstrahl vorgibt.

8. Vorrichtung nach einem der Ansprüche 1 bis 7,
**dadurch gekennzeichnet, dass** im Bereich des jeweils ins Unendliche abgebildeten Beleuchtungs- und Beobachtungsstrahl wenigstens eine Filtereinheit (F1, F2) längs des Beleuchtungs- und/oder Beobachtungsstrahls vorgesehen ist, die schwenkbar und/oder in ihrer Filterwirkung veränderlich ausgebildet und angeordnet ist.

9. Vorrichtung nach einem der Ansprüche 1 bis 8,
**dadurch gekennzeichnet, dass** die Ophthalmoskopierlinseneinheit (1) eine asphärische Linseneinheit ist, die die zweite Zwischenbildebene (ZB2) auf den Augenhintergrund abbildet.

10. Vorrichtung nach Anspruch 9 in Kombination mit einer Vorrichtung zur okulären Kohärenztomographie, kurz OCT, die eine Abbildungsoptik zur Abbildung eines Messstrahls in den Bereich des Augenhintergrundes sowie zur rückwärtigen Abbildung von am Augenhintergrund gestreuter Messstrahlanteile aufweist,
**dadurch gekennzeichnet, dass** die Abbildungsoptik der Vorrichtung zur okulären Kohärenztomographie aus der Ophthalmoskopierlinseneinheit (1) sowie einer Kompensationsoptik (13) besteht,
dass zwischen der Ophthalmoskopierlinseneinheit (1) und der Kompensationsoptik (13) eine Strahlzusammenführungseinheit (12) eingebracht ist, die den Messstrahl und den Beleuchtungs- und Beobachtungsstrahl zur gemeinsamen Abbildung durch die Ophthalmoskopierlinseneinheit (1) zusammenführt.

11. Vorrichtung nach Anspruch 10,
**dadurch gekennzeichnet, dass** die Strahlzusammenführungseinheit (12) als geometrischer oder als dichroitischer Strahlteiler ausgebildet ist.

12. Vorrichtung nach Anspruch 11,
**dadurch gekennzeichnet, dass** der geometrische oder dichroitische Strahlteiler transparent für den Messstrahl und reflektierend für den Beleuchtungs- und Beobachtungsstrahl ist oder umgekehrt.

13. Vorrichtung nach einem der Ansprüche 10 bis 12,
**dadurch gekennzeichnet, dass** die Abbildungsoptik des OCT eine Brennweite besitzt, die der resultierenden Brennweite eines aus der Ophthalmoskopierlinseneinheit (1) und der Kompensationsoptik (13) bestehenden optischen Systems entspricht.

14. Vorrichtung nach einem der Ansprüche 10 bis 13,
**dadurch gekennzeichnet, dass** die Kompensationsoptik (13) folgende Komponenten umfasst: ein telezentrisches Linsensystem (18), das den Messstrahl des OCT ins Unendliche abbildet, ein afokales Galileisystem (18), das den parallel gerichteten Messstrahl in einen fokussierenden Achromaten (15) abbildet, der den Messstrahl in die zweite Zwischenbildebene (ZB2) abbildet.

15. Vorrichtung nach Anspruch 14,
**dadurch gekennzeichnet, dass** zwischen dem fokussierenden Achromat (15) und der zweiten Zwischenbildebene (ZB2) die Strahlzusammenführungseinheit (12) angeordnet ist.

## Revendications

1. Dispositif pour examiner visuellement le fond de l'oeil d'un patient avec :
- un dispositif d'éclairage (L) pour générer un faisceau lumineux ;
- une cartographie d'éclairage optique destinée à projeter le faisceau lumineux sur l'oeil, contenant un dizaine de condenseurs (10) cartographiant un faisceau de lumière sortant du dispositif d'éclairage (L) dans un plan d'image intermédiaire (ZB1), un premier objectif de cartographie (2) qui cartographie le plan d'image intermédiaire (ZB1) à l'infini, un deuxième objectif de cartographie (5) qui cartographie le plan d'image intermédiaire cartographié à l'infini sur un second plan d'image intermédiaire (ZB2), ainsi qu'une unité de lentille d'ophtalmoscopie (1) qui cartographie le deuxième plan d'image intermédiaire (ZB2) sur le fond de l'oeil,
- un procédé pour balayer l'oeil avec le faisceau lumineux (11),
- un dispositif d'observation (9) ;
- une cartographie d'observation optique pour cartographier un faisceau d'observation sur le dispositif d'observation (9) généré sur l'oeil (11) grâce à la réflexion du faisceau lumineux comprenant le faisceau lumineux projeté sur le fond d'oeil dans l'unité de lentille d'ophtalmoscopie (1) cartographiant le second plan d'image intermédiaire (ZB2), le deuxième objectif de cartographie (2) qui cartographie le second plan d'image intermédiaire (ZB2) à l'infini, un faisceau d'observation cartographié à l'infini dans un troisième plan d'image intermédiaire (ZB3) en cartographiant l'objectif (5'), et une troisième lentille de cartographie (8) cartographiant le troisième plan d'image intermédiaire (ZB3) dans le dispositif d'observation (9), et
- un procédé pour bloquer la lumière diffusée provenant du faisceau d'observation,
**caractérisé en ce qu'**un prisme à arête réfringente (3) est prévu le long du faisceau lumineux cartographié à l'infini et un élément de déviation ( 4 ) est prévu le long du faisceau d'observation cartographié à l'infini, et
que le prisme à arête réfringente (3) est agencé de sorte que sa position est modifiable et/ou l'élément de déviation ( 4) est disposé de telle sorte que sa position est modifiable, de sorte qu'un déplacement du faisceau latéral assignable (A) est variable entre le faisceau lumineux et le faisceau d'observation dans le deuxième objectif de cartographie ( 2), et
que les dispositifs de balayage du faisceau d'illumination et les procédés pour bloquer la lumière diffusée de manière réversible sont convertibles entre au moins deux positions de fonctionnement, de manière à ce que, dans une première position de fonctionnement de l'éclairage, le faisceau d'observation sur le fond de l'oeil a une première surface de section transversale du faisceau et dans une deuxième position de fonctionnement de l'éclairage, le faisceau d'observation sur le fond de l'oeil a une seconde surface de section transversale du faisceau, dans lequel les première et deuxième surfaces de section transversale du faisceau sont différentes.

2. Dispositif selon la revendication 1,
**caractérisé en ce que** les procédés de balayage du faisceau lumineux et les procédés de blocage de la lumière diffusée sont sous forme de tambour (6) tournant autour d'un axe qui présente au moins une première et une seconde rangée de fentes (T1, T2), dans lequel respectivement un nombre n ≥ 2 de fentes de dimensions égales est fourni dans chaque cas, à une distance équidistante appariée dans la direction circonférentielle du tambour (6),
- que le faisceau lumineux et le faisceau d'observation passent à travers la rangée de fentes (T1, T2) sont à deux endroits différents dans la direction circonférentielle du tambour (6),
- que la deuxième rangée de fentes (T2) est disposée longitudinalement par rapport à l'axe de rotation près de la première rangée de fentes (T1) dans le tambour (6),
- que la fente dans la seconde rangée de fentes (T2) a une taille différente de celle des fentes de la première rangée de fentes (T1), et
- que par rapport au faisceau d'observation et au faisceau lumineux, le tambour (6) est variable en fonction de son emplacement.

3. Dispositif selon la revendication 2,
**caractérisé en ce que** les fentes (T1, T2) ont une longueur orientée parallèlement à l'axe de rotation et une largeur orientée perpendiculairement à l'axe de rotation et **en ce que** les fentes de la seconde rangée de fentes (T2) ont une largeur supérieure à celle des fentes de la première rangée de fentes (T1).

4. Dispositif selon l'une quelconque des revendications 1 à 3,
**caractérisé en ce que** le long d'un axe optique associé au deuxième objectif de cartographie (2), l'élément de déviation (4) est disposé entre le prisme à arête réfringente (3) et le deuxième objectif d'imagerie (2), et
**en ce que** le faisceau lumineux et le faisceau d'observation présentent au moins un déplacement du faisceau latéral de taille égale (A) de l'axe optique dans la zone située entre l'élément de déviation (4) et le deuxième objectif d'imagerie (2).

5. Dispositif selon l'une quelconque des revendications 2 à 4,
**caractérisé en ce que** les endroits, où le faisceau lumineux et le faisceau d'observation traversent l'au moins une rangée de fentes, sont diamétralement opposés les uns par rapport aux autres dans la direction circonférentielle du tambour (6).

6. Dispositif selon l'une quelconque des revendications 2 à 5,
**caractérisé en ce que** le premier plan d'image intermédiaire (ZB1) est situé à l'endroit où le faisceau lumineux traverse la rangée de fentes du tambour (6), et **en ce que** le troisième plan d'image intermédiaire (ZB3) est situé à l'endroit où le faisceau d'observation traverse la rangée de fentes du tambour (6).

7. Dispositif selon l'une quelconque des revendications 2 à 6,
**caractérisé en ce qu'**à l'intérieur du tambour (6), un élément de déviation de faisceau (7) est prévu pour le faisceau lumineux et un autre élément de déviation de faisceau (7') est prévu pour le faisceau d'observation, où la distance mutuelle fixe un déplacement du faisceau latéral maximal entre le faisceau lumineux et le faisceau d'observation.

8. Dispositif selon l'une quelconque des revendications 1 à 7,
**caractérisé en ce que** dans les zones du faisceau lumineux et du faisceau d'observation respectivement mises en correspondance à l'infini, au moins une unité de filtre (F1, F2) est prévue le long du faisceau lumineux et/ou du faisceau d'observation formé et disposé de sorte qu'elle est pivotable et/ou variable dans son effet de filtrage.

9. Dispositif selon l'une quelconque des revendications 1 à 8,
**caractérisé en ce que** l'unité de lentille d'ophtalmoscopie (1) est une unité de lentille asphérique qui cartographie le second plan d'image intermédiaire (ZB2) sur le fond d'oeil.

10. Dispositif selon la revendication 9 en combinaison avec un dispositif pour une tomographie à cohérence optique (OCT) qui comporte une lentille de cartographie pour cartographier un faisceau de mesure dans la zone du fond d'oeil, ainsi que pour la cartographie inverse des composantes du faisceau de mesure diffusée sur le fond d'oeil, **caractérisé en ce que** la lentille de cartographie de l'appareil de tomographie à cohérence optique (OCT) se compose de l'unité de lentille d'ophtalmoscopie (1) et d'une lentille de compensation (13), et
**en ce qu'**une unité de combinaison de faisceau (12) est introduite entre l'unité de lentille d'ophtalmoscopie (1) et la lentille de compensation (13) qui combine le faisceau de mesure (M), et le faisceau lumineux ainsi que le faisceau d'observation à la cartographie commune à travers l'unité de lentille d'ophtalmoscopie (1).

11. Dispositif selon la revendication 10,
**caractérisé en ce que** l'unité de combinaison de faisceau (12) est conçue comme un séparateur de faisceau géométrique ou dichroïque.

12. Dispositif selon la revendication 11,
**caractérisé en ce que** le séparateur de faisceau géométrique ou dichroïque est transparent pour le faisceau de mesure et réflectif pour le faisceau lumineux et le faisceau d'observation, ou vice versa.

13. Dispositif selon l'une quelconque des revendications 10 à 12,
**caractérisé en ce que** la lentille de cartographie de l'OCT a une largeur focale qui correspond à la longueur focale résultante d'un système optique constitué de l'unité de lentille d'ophtalmoscopie (1) et de la lentille de compensation (13).

14. Dispositif selon l'une quelconque des revendications 10 à 13, **caractérisé en ce que** la lentille de compensation (13) est constituée des composants suivants :
un système de lentille télécentrique (18) qui cartographie le faisceau de mesure à l'infini, un système afocal galiléen (16) qui cartographie le faisceau de mesure orienté parallèlement dans une lentille achromatique de focalisation (15) qui cartographie le faisceau de mesure dans le second plan d'image intermédiaire (ZB2).

15. Dispositif selon la revendication 14,
**caractérisé en ce que** l'unité de combinaison de faisceau (12) est disposée entre la lentille de mise au point automatique (15) et le second plan d'image intermédiaire (ZB2).
